# FASCICULE DE BREVET EUROPEEN

(11) **EP 1 297 116 B1**
(45) Date de publication et mention de la délivrance du brevet: **12.04.2006**
(21) Numéro de dépôt: 01947593.8
(22) Date de dépôt: 22.06.2001
(51) Int. Cl.: C12N 9/20, C07K 1/36, A61K 38/43

(54) **PROCEDE D'ISOLEMENT ET DE PURIFICATION D'UNE PROTEINE**
VERFAHREN ZUR ISOLIERUNG EINES PROTEINS
METHOD FOR ISOLATING AND PURIFYING A PROTEIN

(30) Priorité: 23.06.2000 FR 0008118
(43) Date de publication de la demande: 02.04.2003
(73) Titulaire: Warner-Lambert Company LLC, Morris Plains, NJ 07950 (US); MERISTEM THERAPEUTICS, 63100 Clermont-Ferrand (FR)
(72) Inventeur: BERNA, Patrick, F-91120 Palaiseau (FR); CLEMENT, Christelle, F-63270 Vic le Compte (FR)
(74) Mandataire: Thurgood, Alexander John
(86) Numéro de dépôt international: PCT/FR2001/001985
(87) Numéro de publication internationale: WO 2001/098473

(56) Documents cités:
- EP-A- 0 574 050
- WO-A-94/13816
- WO-A-96/33277
- WO-A-96/38469
- US-A- 3 899 395
- DATABASE CHEMABS [en ligne] CHEMICAL ABSTRACTS SERVICE, COLUMBUS, OHIO, US; JOLIFF, GWENNAEL ET AL: "Secretion of an active recombinant dog gastric lipase from baculovirus-infected insect cells" retrieved from STN Database accession no. 129:188390 CA XP002165073 & BIOTECHNOL. LETT. (1998), 20(7), 697-702, 1998,
- DATABASE CHEMABS [en ligne] CHEMICAL ABSTRACTS SERVICE, COLUMBUS, OHIO, US; CARRIERE, FREDERIC ET AL: "Purification and biochemical characterization of dog gastric lipase" retrieved from STN Database accession no. 116:123655 CA XP002165074 & EUR. J. BIOCHEM. (1991), 202(1), 75-83, 1991,

## Description

La présente invention concerne un procédé d'isolement et de purification d'une protéine d'intérêt en solution, comprenant des étapes d'agrégation partielle et d'adsorption de ladite protéine, notamment à partir d'un milieu complexe, comprenant des lipides et/ou des composés protéiques et/ou des polysaccharides et/ou des pigments et/ou des polyphénols.

### Etat de la technique

Les techniques d'isolement et de purification de protéines impliquent en général une étape de solubilisation de la protéine à isoler, suivie d'une ou plusieurs étapes successives visant à séparer du milieu de départ la protéine d'intérêt à purifier.

La protéine d'intérêt à purifier peut être séparée du milieu dans lequel elle se trouve solubilisée par précipitation. Dans ce type de procédé, l'homme du métier peut avoir recours à l'addition d'un composé précipitant des protéines ajusté à une concentration telle qu'il permet la formation de macro-agrégats moléculaires contenant la protéine d'intérêt, ces macro-agrégats moléculaires étant des assemblages moléculaires d'une taille suffisante pour permettre leur décantation spontanée au sein de la solution. En règle générale, la fraction solide comprenant les macro-agrégats moléculaires est séparée de la fraction soluble par centrifugation, après quoi le culot insoluble contenant la protéine d'intérêt est récupéré.

De telles techniques de séparation de protéines par précipitation sont notamment décrites dans la demande DE 1 642 654, ainsi que dans les brevets US 4,742,159, US-5,169,936, US 4,624,918, US 4,470,969 et US 4,343,735.

En particulier, la demande DE 1.642.654 concerne l'extraction d'une lipase à partir de cultures cellulaires de Rhizopus arrhizus. Il est précisé que la lipase peut être séparée par insolubilisation dans des solvants organiques ou dans des solutions salines concentrées, telles que des solutions de sulfate d'ammonium.

D'autres procédés de séparation de protéines comprennent une ou plusieurs étapes de filtration sur divers supports solides, de telles filtrations visant à retenir majoritairement la protéine à purifier, essentiellement par une action mécanique d'exclusion de taille.

Parmi les supports de filtration décrits dans l'état de la technique, la terre de diatomées a été utilisée dans des procédés de purification de protéines, telles que l'érythropoïétine présente dans l'urine humaine ou encore l'antigène de surface de l'hépatite B (HbS) présent dans un lysat bactérien (voir, FR 2.467.214 et EP 0 480 525).

La protéine d'intérêt peut également être isolée du milieu dans lequel elle est solubilisée par un passage de ce milieu sur un support de chromatographie, destiné à retenir spécifiquement la protéine d'intérêt et ainsi à exclure la majorité du matériel non désirable, tel que des protéines non apparentées.

Parmi les procédés de chromatographie classiquement utilisés, on peut citer notamment la chromatographie d'exclusion, la chromatographie d'échange d'ions, ou encore la chromatographie en phase inverse.

Les techniques de purification de protéines de l'état de la technique nécessitent de procéder à l'isolement de la protéine recherchée en plusieurs étapes distinctes les unes des autres car utilisant des "outils" très différents voire incompatibles entre eux (comme par exemple l'obtention d'un précipitat insoluble suivi d'une chromatographie).

En outre, ces techniques permettent parfois l'obtention de la protéine désirée à un haut degré de pureté et avec un rendement économiquement satisfaisant lorsque ces protéines sont excrétées sous forme soluble dans des milieux peu riches en protéines et / ou en métabolites secondaires tels que certains fluides naturels comme l'urine ou encore des milieux de culture bactériens ou de lignées cellulaires eucaryotes.

La purification d'une protéine d'intérêt se révèle toutefois plus délicate et plus difficile à mettre au point lorsque le milieu initial et comporte un grand nombre de métabolites secondaires, dans lequel la, protéine d'intérêt est sous forme soluble, comprend de nombreuses protéines indésirables ainsi que des composés de nature lipidique, ou polysaccharidique, des polyphénols ou encore certains pigments constitués de structures aromatiques liées à des chaînes grasses, tels que les pigments xanthophylles.

Ceci est le cas notamment lorsque la protéine d'intérêt doit être purifiée à partir d'un matériel végétal d'une plante à forte teneur en lipides, à forte teneur en sucres, ou encore à forte teneur en polyphénols, comme c'est le cas pour le maïs ou le tabac.
La présente invention permet de pallier efficacement les inconvénients rencontrés dans l'art antérieur, notamment par le fait que ce nouveau procédé de purification ne comprend pas une insolubilisation totale de la protéine conduisant à un précipitat décantant spontanément mais simplement en une étape d'agrégation partielle et d'adsorption de la protéine recherchée sur un support solide.
Ce procédé peu onéreux permet d'obtenir d'excellents résultats dans la purification de protéines à partir de milieux complexes et notamment à des niveaux industriels car pouvant être effectué de manière continue, c'est-à-dire sans interruption dans le processus de purification, à l'inverse des techniques décrites dans l'art antérieur. Ainsi, le procédé de l'invention présente l'avantage de permettre la purification d'une protéine d'intérêt en un nombre d'étapesxréduit. En outre, ce procédé permet d'intervenir très tôt dans la purification de molécules car il est adapté à la purification de protéines à partir de milieux dits complexes comprenant des dérivés protéiques, lipidiques, polysaccharidiques ou autres.
On constate par ailleurs, que selon le procédé préféré de la présente invention, on élimine de manière surprenante environ 97% des protéines non souhaitées, et ce de manière sélective, environ 70 % des lipides et plus de 99% des amidons présents dans le milieu complexe de départ.

### Résumé de l'invention

L'invention a pour objet un procédé d'isolement et de purification d'une protéine d'intérêt, comprenant des étapes d'agrégation partielle et d'adsorption de ladite protéine sur un support solide, réalisée de manière simultanées; ladite étape d'agrégation partielle comprenant l'introduction dans ladite solution d'un agent précipitant qui génère dés assemblages moléculaires de ladite protéine de petite taille, restant en suspension dans la solution et non susceptibles de décanter spontanément (micro-agrégats) et qui sont adsorbés sur ledit support solide. Le procédé de l'invention est avantageusement mis en oeuvre lors de purification de protéines d'intérêt à partir d'un milieu complexe.

Le procédé d'isolement et de purification de l'invention est caractérisé en ce qu'il comprend une étape au cours de laquelle un milieu complexe, comprenant la protéine d'intérêt à purifier ainsi qu'un support solide susceptible d'en permettre l'adsorption, est mis en présence d'un agent capable de provoquer la précipitation de cette protéine en solution, la protéine d'intérêt étant ainsi précipitée puis adsorbée sur le support solide sans qu'il y ait formation substantielle de macro-agrégats dans la solution.
Le procédé selon l'invention est en particulier caractérisé en ce que la cinétique de l'étape d'agrégation partielle est modifiée par la cinétique de l'étape d'adsorption en ce sens que la cinétique d'adsorption favorise la formation des micro-agrégats et défavorise la formation de macro-agrégats.

La présente invention est illustrée, sans pour autant être limitée, par les tableaux et figures suivantes:
Tableau 1 : données brutes de l'influence de la quantité de sulfate d'ammonium sur le phénomène d'adsorption sur terre de diatomées de la lipase gastrique recombinante exprimée dans le maïs.
Tableau 2 : données brutes de l'influence de la quantité de sulfate de sodium sur le phénomène d'adsorption sur terre de diatomées de la lipase gastrique recombinante exprimée dans le maïs
Figure 1: influence de la quantité de sulfate d'ammonium sur le phénomène d'adsorption sur terre de diatomées de la lipase gastrique recombinante exprimée dans le maïs.
Figure 2 : influence de la quantité de sulfate de sodium sur le phénomène d'adsorption sur terre de diatomées de la lipase gastrique recombinante exprimée dans le maïs.
Figure 3 : influencé de la quantité de polyéthylène glycol (PEG 4000) sur le phénomène d'adsorption sur terre de diatomées de la lipase gastrique recombinante exprimée dans le maïs.
Figure 4 : influence du type de support solide sur l'adsorption de la lipase gastrique recombinante en présence de sulfate d'ammonium.
Figure 5 : influence de la quantité de sulfate d'ammonium sur le phénomène d'adsorption de différents types de protéines sur terre de diatomées.
Figure 6 : schéma général de la purification de la lipase gastrique de chien recombinante selon le procédé de l'invention, à partir de grains de maïs ou de feuilles de tabac lyophilisées.

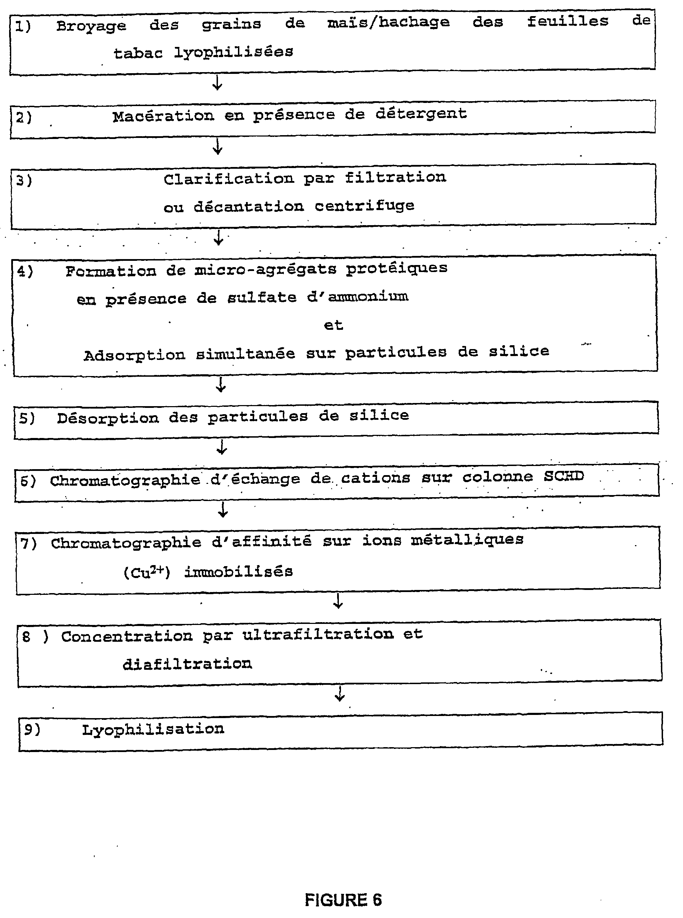

Les étapes successives du procédé de l'invention sont résumées à la figure 6 et les légendes correspondantes sont intégrées à la présente description.

### Description détaillée de l'invention

Les inventeurs ont donc mis en évidence un moyen de purifier une protéine d'intérêt en solution, notamment à partir d'un milieu complexe, par l'addition dans le milieu d'un agent précipitant et d'un support solide adsorbant, permettant ainsi l'adsorption de la protéine à purifier directement sur le support solide en évitant la formation substantielle de macro-agrégats de la protéine dans la solution.
L'ajout de l'agent précipitant peut être simultané ou suivre dans un délai plus ou moins long l'introduction du support solide.

Dans des milieux complexes, et notamment des milieux contenant des lipides et/ou des polysaccharides et/ou des polyphénols et/ou des pigments à chaînes grasses, les étapes classiques de purification par chromatographie sont rendues très inefficaces. Plus particulièrement, les inventeurs ont observé que dans de telles solutions, la protéine d'intérêt à purifier n'est pas sélectivement retenue par les différents supports chromatographiques testés.

L'absence de fixation de la protéine à purifier à partir d'un milieu complexe sur un support de chromatographie peut être expliquée par deux phénomènes qui peuvent apparaître conjointement :
- d'une part, les composés indésirables de la solution complexe sont adsorbés sur le support chromatographique et saturent ainsi la majorité des sites, qui ne sont dès lors plus accessibles pour la fixation de la protéine d'intérêt;
- d'autre part, certains composés, comme les polysaccharides ou les lipides, sont susceptibles d'environner les molécules de protéine à purifier, et ainsi d'empêcher tout contact de la protéine à purifier avec le support de chromatographie.

En solution aqueuse, une protéine solubilisée est hautement hydratée, c'est-à-dire que les groupes ioniques présents à la surface de la protéine attirent et fixent de nombreuses molécules d'eau par l'intermédiaire de liaisons faibles (liaisons hydrogène, liaisons de Van der Waals).

Lorsqu'un agent précipitant les protéines, tel que le sulfate d'ammonium, est ajouté à la solution contenant la protéine d'intérêt; les ions de l'agent précipitant attirent les molécules d'eau et les rendent ainsi inaccessibles à la protéine initialement en solution.

En l'absence d'un nombre suffisant de liaisons faibles entre les molécules protéiques en solution et les molécules d'eau avoisinantes, les molécules de protéine ont tendance à interagir entre elles et commencent à s'agréger. Classiquement, la concentration d'agent précipitant est ajustée de sorte que la molécule protéique soit fortement dépourvue de liaisons faibles avec les molécules d'eau avoisinantes et que les molécules protéiques initialement en solution interagissent de telle manière à former des macro-agrégats ayant une taille suffisante permettant leur décantation spontanée dans la solution et leur récupération, en général par centrifugation, sous forme d'une fraction solide retrouvée intégralement dans le culot.

Lorsque les inventeurs ont tenté de séparer par précipitation à l'aide du sulfate d'ammonium une protéine, la lipase gastrique de chien, d'un milieu complexe, un extrait végétal, un phénomène inattendu a été observé. En effet, une solution triphasique a été obtenue après centrifugation de la fraction rendue insoluble du fait de l'addition du sulfate d'ammonium. Cette solution triphasique comprenait une phase solide retrouvée en culot après centrifugation, et deux phases liquides sans interface nette, la protéine à purifier étant retrouvée au sein de la phase liquide supérieure de nature lipophile. L'absence d'interface nette constituée entre la phase inférieure liquide et la phase supérieure liquide était de nature à rendre difficile, sinon impossible avec des rendements acceptables ou satisfaisants, la récupération de la fraction liquide supérieure contenant la protéine à purifier. Dans tous les cas, la récupération de la phase liquide supérieure contenant la protéine d'intérêt entraînait une perte importante de la protéine à purifier, et diminuait considérablement l'intérêt économique d'un tel procédé de purification.

Les inventeurs ont donc cherché un moyen efficace et pratique d'isoler une protéine d'intérêt dans un milieu complexe.
C'est pourquoi, un premier objet de l'invention se rapporte à un procédé d'isolement et/ou de purification d'une protéine d'intérêt en solution comprenant des étapes d'agrégation partielle et d'adsorption de ladite protéine sur un support solide, ladite étape d'agrégation partielle comprenant l'introduction dans ladite solution d'un agent précipitant qui génère des assemblages moléculaires de ladite protéine de petite taille, non susceptibles de décanter spontanément et qui sont adsorbés sur ledit support solide.
En effet, au contraire des procédés de précipitation évoqués ci-dessus, le procédé de l'invention comporte une étape au cours de laquelle un agent précipitant est ajouté à un milieu comprenant la solution contenant la protéine d'intérêt à purifier ainsi qu'un support solide de préférence non greffé de ligands spécifiques susceptibles d'interagir avec la protéine d'intérêt.
De cette façon, les inventeurs sont d'avis que la protéine d'intérêt qui commence à former des assemblages moléculaires de petites tailles (micro-agrégats) en présence de l'agent précipitant peut être aussitôt adsorbée sur le support. En particulier, ledit support semble favoriser la sortie rapide de la protéine de la solution en adsorbant ces micro-agrégats de protéine ou même des molécules isolées de celle-ci avant la formation d'une fraction solide susceptible d'être décantée (macro-agrégats).
Ainsi, selon les inventeurs, le procédé de l'invention est caractérisé en ce que les assemblages moléculaires de ladite protéine sont substantiellement formés d'agrégats protéiques de petite taille restant en suspension dans la solution (micro-agrégats).
Par ailleurs, l'agrégation partielle et l'adsorption de ladite protéine sont simultanées. En effet, les inventeurs estiment que la présence du support solide dans la solution contenant la protéine d'intérêt au même moment que l'agent précipitant viendrait modifier la cinétique de la réaction d'agrégation de la protéine, au profit d'une sortie immédiate des molécules de protéine (micro-agrégats déjà formés et/ou molécules isolées) de la solution par adsorption. Cette modification de cinétique ayant pour résultat de défavoriser la formation d'assemblages moléculaires importants (macro-agrégats) et donc de réaliser la purification voulue au moyen d'une étape d'agrégation partielle.

L'expression 'micro-agrégats' se réfère à des assemblages moléculaires contenant la protéine d'intérêt à purifier, dont la taillé est suffisamment petite pour que ces derniers restent en suspension dans la solution. Ainsi ces assemblages moléculaires ne sont pas susceptibles de décanter spontanément.

Le procédé de l'invention présente des avantages considérables par rapport aux procédés de purification de protéines faisant appel à une précipitation. D'une part, il permet d'adsorber la protéine d'intérêt sur un support en une seule étape simple pouvant être intégrée dans un processus industriel, et plus particulièrement en un processus industriel en continu. D'autre part, il permet un ajustement optimal des concentrations d'agent précipitant ajoutées dans le milieu comprenant la protéine à purifier et plus particulièrement une diminution de la quantité d'agent précipitant nécessaire pour faire " sortir " la protéine d'intérêt de la solution en l'adsorbant sur le support solide.

Les protéines ainsi adsorbées peuvent par ailleurs être ensuite facilement désorbées du support solide selon les techniques classiques et en l'absence de l'agent précipitant, afin d'être récupérées et éventuellement soumises à d'autres étapes de purification, par exemple par chromatographie.

### Protéines d'intérêt susceptibles d'être purifiées par le procédé de l'invention

Le procédé selon l'invention est particulièrement adapté à la purification de protéines à partir de milieux complexes, c'est-à-dire de milieux comprenant seuls ou en combinaison, des composés protéiques c'est-à-dire des protéines ou des parties de protéines non apparentées à la protéine d'intérêt, des polysaccharides, des composés lipidiques, des polyphénols et/ou des pigments notamment des pigments à chaîne grasse comme les xanthophyllines. Ainsi, le procédé de l'invention peut être mis en oeuvre pour la purification de protéines à partir de matériel d'origine animale, bactérienne, virale ou fongique et avantageusement à partir de matériel biologique tel que le sérum foetal, le plasma sanguin ou encore à partir de matériel végétal, et plus particulièrement à partir de matériel végétal riche en lipides, polysaccharides, polyphénols et/ou pigments à chaîne grasse, tels que les végétaux oléagineux, protéagineux, les végétaux à forte teneur en polysaccharides, ou encore les végétaux à forte teneur en pigments.

De manière tout à fait préférée, le procédé de l'invention est utilisé pour purifier une lipase gastrique recombinante et en particulier une lipase gastrique recombinante de chien exprimée dans un végétal transgénique, tel que le maïs, le tabac, la tomate, le colza, le soja, le riz, la pomme de terre, la carotte, le blé, forge, le tournesol, la laitue ou encore l'avoine.

Dans un mode de réalisation avantageux du procédé selon l'invention, la lipase gastrique recombinante de chien est exprimée dans un végétal transgénique, conformément à l'enseignement de la demande de brevet PCT FR 96/00606 publiée sous le numéro WO 96/33277, et dont le contenu est incorporé par référence à la présente demande.

Plus particulièrement, la lipase gastrique de chien recombinante, telle qu'isolée par le procédé selon l'invention, est pure à au moins 90% par rapport à la surface d'un pic d'absorbance UV à 230 nm par rapport à la surface totale des pics d'absorption, de préférence à au moins 92% et de manière tout à fait préférée à au moins 95%.

Le procédé de la présente invention n'est toutefois pas limité à l'isolation de la lipase gastrique de chien recombinante. Aussi, un autre objet de l'invention se rapporte-t-il à une protéine d'intérêt purifiée par le procédé précédemment décrit. En plus de la lipase gastrique de chien, plusieurs types de protéines d'intérêt et en particulier des lipases gastriques d'extraction ou recombinantes peuvent en effet être séparées de divers milieux complexes par l'intermédiaire de ce procédé avec des degrés de pureté sensiblement équivalents. Quelques-unes des caractéristiques de la protéine à purifier peuvent être prises en compte par l'homme du métier pour ajuster les paramètres du procédé de l'invention comprenant la masse moléculaire, les propriétés de surface ainsi que le point isoélectrique.

### a) Masse moléculaire

Les résultats des travaux réalisés ont d'abord permis de démontrer que des protéines de masse moléculaire très différente pouvaient être isolées et purifiées par l'intermédiaire du procédé de l'invention. A titre d'exemple, des protéines de masse moléculaire entre environ 20 et 200 kD ont pu être séparées par adsorption sur terre de diatomées suite à l'introduction d'une concentration optimale de sulfate d'ammonium dans la solution où se trouvait ces protéines. Les résultats préliminaires obtenus jusqu'à présent ne semblent cependant pas indiquer de relation de proportionnalité directe entre la masse moléculaire de la protéine à séparer et la concentration d'agent précipitant.

### b) Propriétés de surface et point isoélectrique

Les propriétés de surface de la protéine à précipiter ont une influence très dominante sur sa solubilité. En effet, plus il est facile de séparer les molécules d'eau fixées à la surface de la protéine, plus il sera facile de l'agréger partiellement. Il est donc important pour l'homme du métier de prendre en compte la présence de groupements hydrophobes, hydrophiles se trouvant normalement à la surface de la protéine d'intérêt lorsqu'il est nécessaire d'en effectuer la purification selon le procédé de l'invention. On peut par exemple considérer que la présence de groupements hydrophiles à la surface de la protéine nécessitera globalement une concentration d'agent précipitant plus élevée pour l'agréger partiellement que si cette dernière comporte des groupements hydrophobes lui conférant une solubilité de base plus faible. L'autre caractéristique de la protéine à purifier devant être regardée par l'homme du métier pour ajuster les paramètres du procédé de l'invention est le point isoélectrique de la protéine d'intérêt. En effet, cette caractéristique semble également avoir une influence sur la solubilité de la protéine en solution et doit donc être prise en compte lors de la mise en oeuvre du procédé de la présente invention.
Les caractéristiques mentionnées précédemment permettent une appréciation globale de la solubilité de la protéine d'intérêt à purifier, les paramètres du procédé peuvent ensuite être ajustés plus précisément et plus rapidement en fonction de la solubilité supposée de la protéine à isoler.

### Paramètres principaux du procédé de l'invention

### a) Agents précipitants

Selon le procédé de la présente invention, plusieurs types d'agents précipitants différents peuvent être utilisés. L'homme du métier pourra choisir parmi les sels organiques, des sels inorganiques ou encore des composés de type polyalkylène glycol et préférentiellement le polyéthylène glycol. A titre d'exemple de sels organiques on peut citer l'acétate d'ammonium, et de sels inorganiques on peut citer le sulfate d'ammonium
ou le sulfate de sodium. Des sels cosmotropiques ainsi que des polyots, des carbohydrates, et des composés tels que les méthylpentanediol (MPD) peuvent également être utilisés dans le procédé selon l'invention.

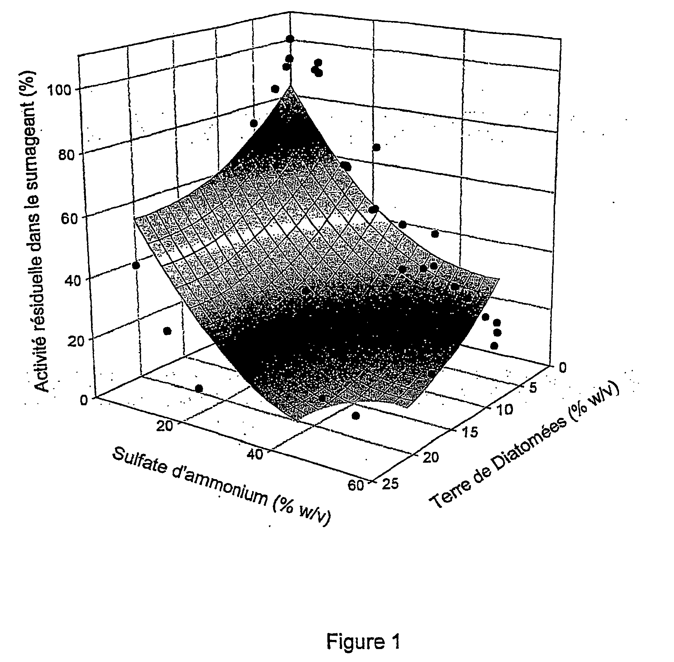

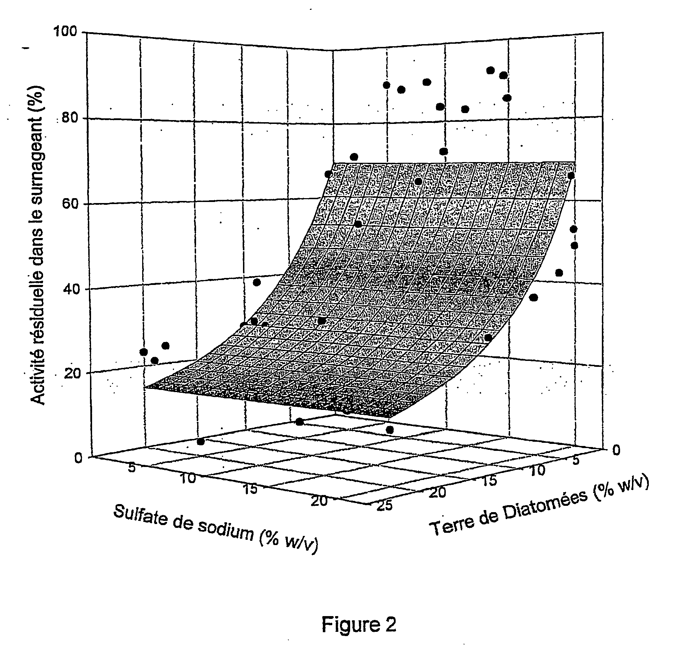

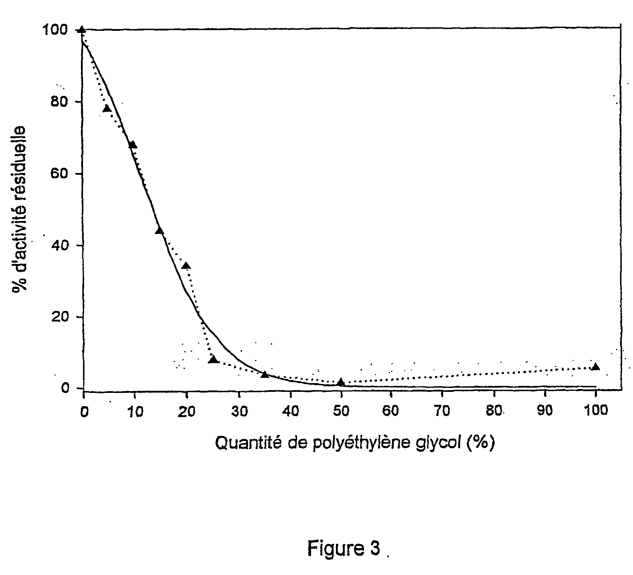

Ainsi qu'illustré aux figures 1, 2 et 3 de la présente demande, la lipase gastrique recombinante de chien exprimée dans le maïs a pu être purifiée en utilisant 3 agents précipitants différents : le sulfate d'ammonium, le sulfate de sodium ainsi que polyéthylène glycol. Le choix de l'agent précipitant le mieux approprié à la précipitation de la protéine que l'on souhaite isoler peut être facilement effectué par l'homme du métier.

En ce qui concerne la concentration d'agent précipitant nécessaire pour isoler une quantité significative de protéine du milieu concerné, les essais effectués jusqu'à présent semblent démontrer que cette concentration varie en fonction de l'agent précipitant utilisé. A titre d'exemple, les inventeurs ont mis en oeuvre le procédé selon l'invention afin de purifier à homogénéité une lipase gastrique de chien recombinante à partir d'un matériel végétal tel que des grains de maïs ou des feuilles de tabac. Pour la purification de cette lipase gastrique de chien recombinante à partir d'une solution complexe (un extrait végétal), des concentrations de sulfate d'ammonium se situant entre 10 et 60 % en poids/volume, de préférence entre 15 et 45 % en poids/volume et préférentiellement entre 15 et 30% en poids/volume ont permis de récupérer la plus grande concentration de lipase. En revanche, ces concentrations sont plus faibles lorsque le sulfate de sodium est utilisé. Dans ce dernier cas, les concentrations de sulfate de sodium varient de façon préférée entre 10 et 30 % en poids/volume . Lorsqu'il s'agit d'utiliser le polyéthylène glycol (PEG 4000), les concentrations optimales se situent entre 20 et 40 % et de façon préférée entre 25 et 35 % en poids/volume.

La concentration d'agent précipitant à utiliser est non seulement fonction de sa nature même mais également, dans une certaine mesure, de la nature et de la concentration du support solide adsorbant mis en oeuvre dans le procédé de l'invention.

### b) Supports adsorbants

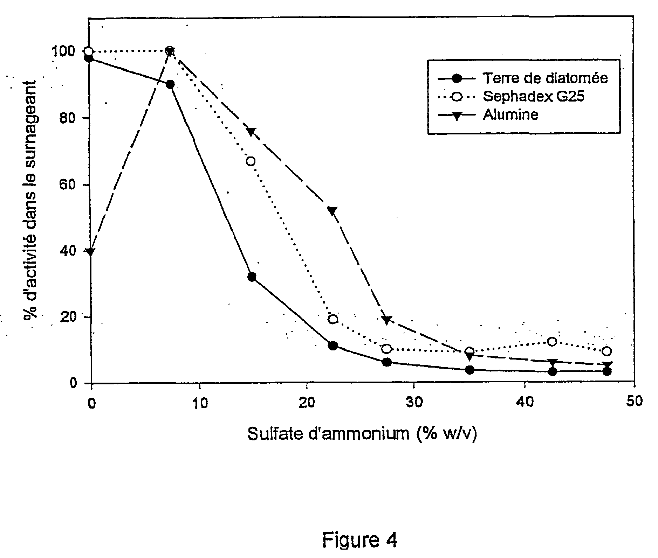

Plusieurs types de supports solides peuvent être utilisés afin d'adsorber la protéine que l'on cherche à purifier. D'une manière générale, le support d'adsorption est un support solide non greffé artificiellement de ligands spécifiques susceptibles d'interagir avec la protéine d'intérêt. Par ligands spécifiques il faut entendre tout ligand capable d'établir une liaison d'affinité ou de type hydrophobe ou donneur-accepteur d'électron(s) avec la protéine voulue. Le support solide est donc plus particulièrement choisi parmi les supports organiques ou inorganiques. A titre d'exemple de supports inorganiques on peut citer des supports à base de silice tel que du verre micro-poreux, du gel de silice ou à base d'oxydes de métaux, de terre de diatomées, d'alumine, des perlites ainsi que des céramiques ou des zéolites. Parmi les supports organiques on peut citer à titre d'exemple les supports à base de dextran, d'agarose, de polyacrylamide, de polystyrène divynylbenzène, de métacrylate, de nylon ou de cellulose. Tel qu'illustré à la figure 4, plusieurs types de supports, notamment de supports très hydrophiles, peuvent être utilisés de façon efficace pour adsorber la lipase gastrique recombinante en présence de sulfate d'ammonium. L'utilisation de 3 supports différents a été étudiée : la terre de diatomées, l'alumine et la résine Sephadex G25. Tel que démontré à la figure 4, chacun de ces supports s'est comporté essentiellement de la même façon dans des expériences d'adsorption de lipase gastrique en présence de sulfate d'ammonium.

Un support solide préféré selon l'invention est constitué par la terre de diatomées. Utilisée en présence de sulfate d'ammonium, de sulfate de sodium ou de polyéthylène glycol, la terre de diatomées a permis, dans les 3 cas, l'adsorption de la quasi totalité de la lipase gastrique recombinante se trouvant dans une solution complexe.

En ce qui concerne la quantité de support solide nécessaire à l'adsorption d'une concentration optimale de protéine à purifier, cette quantité peut évidemment varier en fonction de la nature du support solide utilisé. L'homme du métier pourra facilement déterminer la quantité optimale de support solide en effectuant par exemple un étalonnage préliminaire de la protéine à purifier à des concentrations variables de supports solides.

Plus particulièrement, en ce qui concerne la terre de diatomées, il semble que les quantités optimales permettant la purification d'une concentration acceptable de protéine se trouvant en solution se situe entre 1 et 30 % en poids/volume, plus préférablemént entre 1 et 3 % en poids/volume. De manière préférée, la terre de diatomées peut être incluse dans un filtre de type filtre à cadre. L'utilisation de ce type de filtre peut entre autre permettre le développement d'un procédé de purification en continu. Dans ce contexte, une concentration appropriée d'agent précipitant est introduite et dissoute dans le milieu complexe (à partir duquel la protéine d'intérêt est purifiée) suivi de façon quasi simultanée par l'introduction de la terre de diatomées. La terre de diatomées est maintenue en suspension dans le milieu complexe pendant une période suffisante pour permettre l'adsorption maximale de protéine. La suspension est ensuite passé à travers un filtre à cadre comportant au besoin une pré-couche de terre de diatomées.
Dans le cas de protéines qu'il ne serait pas possible d'isoler en ajustant les paramètres de base du procédé de l'invention, notamment le choix et la concentration de l'agent précipitant et du support solide, des paramètres a priori optionnels comprenant entre autres l'utilisation de réactifs particuliers tels les détergents ou encore l'ajustement du pH devraient alors être pris en compte.
L'homme du métier pourra toutefois avoir recours à des détergents ou encore à des variations de pH afin de mettre en place dans le milieu complexe les conditions d'isolation appropriées.

### Paramètres additionnels facultatifs du procédé de l'invention

### a) Utilisation de détergents

L'utilisation de détergents dans le procédé de la présente invention, bien que généralement optionnelle, peut s'avérer intéressante et utile pour modifier la sélectivité du procédé. En effet, le détergent peut contribuer à modifier les interactions entre les molécules présentes dans le milieu complexe et ainsi influencer l'adsorption sur le support solide de certaines protéines voulues. L'utilisation de plusieurs types de détergents peut être envisagée. A titre d'exemple, on citera les détergents de type Triton X100 et Tween 20.

Dans une des réalisations préférentielles du procédé de l'invention, lorsqu'il s'agit de purifier la lipase gastrique de chien à partir d'un milieu complexe, la protéine est adsorbée sur terre de diatomées qui est ensuite rincée avec un tampon glycine (50 mM) à pH acide en présence d'un détergent. Le détergent préféré dans cette réalisation de l'invention est un détergent non chargé, de type Triton X100 ou encore BRIJ 35.

### b) Influence du pH

Le pH est un paramètre qui peut s'avérer important pour la mise en oeuvre de certaines réalisations préférentielles du procédé de l'invention. En effet, il est possible de modifier le pH du milieu complexe à partir duquel la protéine d'intérêt est isolée afin de modifier les conditions de solubilité de cette protéine en solution.

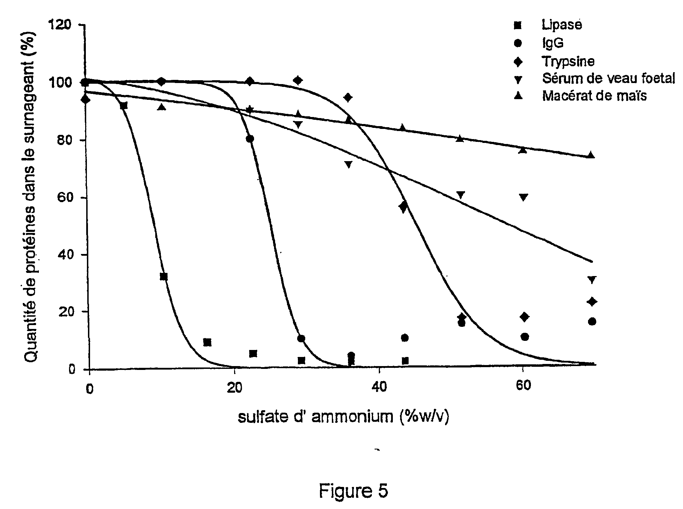

La gamme de pH utilisée dans le cadre du procédé de la présente invention est considérable et peut généralement varier de 2 à 10. A titre d'exemple, la lipase gastrique recombinante a été isolée à pH 3, la trypsine et des IgG ont été isolées à pH 7. Les mêmes gammes de pH sont également envisageables dans le cas de mélanges complexes à partir desquels des protéines doivent être isolées. A titre d'exemple, l'isolation des protéines se trouvant dans un sérum de veau foetal a été effectuée à pH 7 (voir figure 5). En revanche, des protéines se trouvant dans un macérat de maïs ont été isolées à pH 3.

De façon plus générale, l'homme du métier peut avoir recours à des essais préliminaires dans lesquels les paramètres importants (type et concentration de l'agent précipitant, support solide) et éventuellement les paramètres plus optionnels (ajout de réactifs supplémentaires, pH) du procédé sont variés un par un jusqu'à ce que les conditions optimales soient atteintes.
Le procédé selon l'invention se rapporte également à une étape de désorption de la protéine d'intérêt du support solide. Cette étape a lieu en l'absence de l'agent précipitant.

Après rinçage, la protéine adsorbée est désorbée par élution dans un tampon à pH acide ne comportant pas l'agent précipitant. La totalité de l'éluat de désorption est récupérée.

Dans un mode de réalisation particulier de l'invention et de manière facultative, la solution de désorption est ensuite soumise à une étape de filtration. Les plaques à membranes filtrantes ont avantageusement un seuil de rétention compris entre 5 et 40 micromètres et de manière tout à fait préférée un seuil de rétention de 10 micromètres:
Une fois les étapes d'adsorption et de désorption réalisées, l'éluat de l'étape de désorption, ou le filtrat de l'étape facultative de filtration fine, est de préférence mais non obligatoirement soumis à une ou plusieurs étapes de purification ultérieures, afin d'obtenir un produit final purifié. Quelques-unes des différentes étapes ultérieures de purification pouvant être mises en oeuvre par l'homme du métier sont décrites ci-dessous.

### Étapes ultérieures facultatives de purification

Ces étapes de purification ultérieures sont choisies par l'homme du métier en fonction de la nature de la protéine d'intérêt à purifier (chargée positivement ou négativement, hydrophile ou hydrophobe, riche en histidine, etc.).

### a) Chromatographie

L'éluat de l'étape de désorption, ou le filtrat de l'étape facultative de filtration fine, peut par exempte subir une ou plusieurs étapes de Chromatographie telle que chromatographie d'échange d'ions, chromatographie d'exclusion de taille, chromatographie d'interaction hydrophobe, chromatographie d'affinité sur ions métalliques immobilisés, chromatographie d'affinité ou encore chromatographie à haute performance en phase liquide (HPLC). Cette étape peut alternativement comporter une étape de passage de la protéine d'intérêt désorbée du support solide sur un support chromatographique d'échange de cations.

A titre d'exemple particulier selon l'invention, pour la purification de la lipase gastrique de chien recombinante, la solution de désorption est d'abord diluée si nécessaire afin d'en diminuer la force ionique et ainsi favoriser l'adsorption des substances d'intérêt sur un échangeur d'ions. La solution est ensuite facultativement soumise à une étape de filtration fine, puis chargée sur une colonne de chromatographie d'échange de cations (de type S céramic Hyper D, commercialisée par BIOSEPRA). La colonne chargée subit successivement deux lavages: un premier lavage dans un tampon glycine (50 mM) à pH 3 en présence de détergent (TritonX100, 1 mM) afin d'éliminer les éventuels lipides qui seraient adsorbés sur la colonne et un second lavage dans un tampon glycine (50 mM) à pH 3 afin d'éliminer le détergent résiduel. La colonne est enfin éluée à l'aide d'un tampon acétate/acide acétique (50 mM) à pH 4, ces conditions particulières permettant de charger directement l'éluat résultant sur une autre colonne de chromatographie, sans dialyse ou diafiltration préalable.

L'éluat de sortie de la colonne de chromatographie d'échange de cations peut ensuite être chargé sur une colonne de chromatographie à résine d'affinité sur ions métalliques immobilisés (IMAC). De manière préférée, le gel chromatographique est chargé en cuivre (Cu II). La colonne est soumise à un lavage dans un tampon identique au tampon de chargement, puis éluée à l'aide d'un tampon glycine (10 mM) en présence d'acétate d'ammonium à pH approprié.
Dans l'exemple particulier de la purification de la lipase gastrique de chien, qui est constituée d'une glycoprotéine de 49 kDa contenant 13% de carbohydrates et porte 14 résidus d'histidine, cette caractéristique structurale ayant été mise à profit lors de l'une des étapes particulières de chromatographie du procédé de l'invention. En effet, des résidus histidine exposés à la surface de cette protéine ont la propriété de se fixer par l'intermédiaire de liaisons de coordination à certains ions métalliques immobilisés sur les supports chromatographiques, tels que des ions nickel (Ni II) ou cuivre (Cu II). De tels supports chromatographiques comprenant des ions métalliques immobilisés sont par exemple les colonnes de type IMAC précédemment mentionnées et utilisées dans les exemples 1 et 2. Ainsi, pour la purification de la lipase gastrique de chien l'élution sur la colonne de chromatographie IMAC a été réalisée à l'aide du tampon glycine et en présence d'acétate d'ammonium à pH 3. La présence d'acétate de sodium dans le tampon d'élution est avantageuse, en ce que l'activité enzymatique de la lipase gastrique recombinante purifiée est moins affectée en présence de ce composé volatil qu'en présence d'un tampon classique de chlorure de sodium du fait d'une concentration saline moins grande, et constitue par ailleurs des conditions plus favorables pour une lyophilisation ultérieure de l'éluat contenant la lipase gastrique de chien recombinante.

L'élution a été conduite à un pH fortement acide, ceci étant vraisemblablement dû au fait d'un pKa du site d'interaction de la lipase gastrique recombinante avec le support anormalement bas, inférieur à 4.

### b) Filtration

La protéine ainsi purifiée peut ensuite être soumise à une ou plusieurs étapes d'ultrafiltration et/ou de diafiltration additionnelles, notamment afin de concentrer la solution protéique mais également afin de placer la protéine d'intérêt dans des conditions physico-chimiques de stabilité nécessaires à une étape ultérieure de lyophilisation.

L'étape d'ultrafiltration et/ou de diafiltration peut être indifféremment frontale, tangentielle ou spiralée.

Dans un mode particulier de réalisation du procédé selon l'invention, il s'agit d'une étape d'ultrafiltration frontale sur membrane de type polyéther sulfone d'un seuil de coupure de 30 kDa, ce qui permet de concentrer l'éluat d'un facteur d'au moins 10. Le filtrat résultant est ensuite soumis à deux étapes de diafiltration permettant une dilution d'un facteur 100 de la concentration saline du filtrat et un ajustement du pH à une valeur compatible avec une absence de dénaturation de la protéine recombinante purifiée.

La solution ultrafiltrée puis diafiltrée peut en outre subir une étape de filtration additionnelle destinée à éliminer les éventuelles bactéries présentes, le filtre ayant alors un diamètre moyen de pore de 0,22 µm.
Le procédé selon l'invention peut également comporter une étape de séchage de la solution de protéine purifiée. Cette étape de séchage peut ainsi être réalisée notamment par lyophilisation ou atomisation de la protéine purifiée, selon des techniques bien connues de l'homme du métier.

### Purification de protéines à partir de végétaux (étapes préliminaires)

On aura avantageusement recours au procédé de purification selon l'invention afin d'isoler une protéine d'intérêt produite dans les plantes comme le maïs, le tabac, la tomate, le colza, le soja, le riz, la pomme de terre ou encore la carotte.

En particulier, le procédé de l'invention est particulièrement adapté à la purification de protéines recombinantes exprimées dans des végétaux. C'est pourquoi l'invention se rapporte également à un procédé caractérisé en ce qu'il comporte une première étape de broyage des graines ou de hachage des feuilles, suivi d'une étape de clarification par filtration ou centrifugation.

Les étapes préliminaires principales qui peuvent être envisagées avant la mise en oeuvre du procédé de l'invention sont décrites ci-dessous.

### a) Extraction

De manière préférée, le procédé de purification selon l'invention peut comprendre une première étape consistant à extraire la majorité des protéines à partir du matériel végétal brut, en particulier d'un matériel végétal issu d'une plante transgénique exprimant la protéine recombinante d'intérêt dont la purification est recherchée.

Dans le cas du maïs, l'extraction protéique est réalisée à partir d'un broyat de graines obtenu à partir de graines broyées sur des grilles de diamètre de 1 à 3 mm.

Dans le cas d'une extraction à partir de feuilles de tabac, les feuilles sont facultativement lyophilisées, puis broyées jusqu'à l'obtention d'une poudre.

### b) Macération

La poudre issue de ce premier traitement du matériel végétal peut être macérée dans un tampon acide en présence dé détergent, le tampon étant facultativement additionné d'agent chélatant tel que l'EDTA.

Tout type de détergent peut être mis en oeuvre au cours de l'étape de macération, de manière à solubiliser la majorité la protéine d'intérêt présente dans le broyat de départ, en particulier la poudre de matériel végétal décrite ci-dessus.

Avantageusement, on aura recours à l'utilisation d'un détergent non chargé, c'est-à-dire un détergent qui ne sera pas susceptible de se fixer sur des supports de chromatographie lors d'éventuelles phases ultérieures de purification de la protéine d'intérêt.

Seront donc préférés des détergents comme le TritonX100 ou encore le BRIJ 35, de préférence utilisés à une concentration égale à 10 fois la concentration micellaire critique (CMC).

En conséquence, le TritonX100 sera, au cours de l'étape de macération, de préférence utilisé à une concentration comprise entre 0,5 mM et 2 mM, et de préférence à une concentration de 1 mM.

L'étape de macération a une durée comprise entre 5 et 20 heures, et est de préférence d'environ 15 heures, par exemple une durée de 16 heures. Durant l'étape de macération, le pH est avantageusement compris entre 2,5 et 4, et est préférentiellement ajusté à 3.

### c) Clarification

L'étape de macération peut être suivie d'une étape de clarification destinée à éliminer les particules insolubles de grande taille, telles que des débris issus du matériel végétal initial, les agrégats etc. La clarification peut être réalisée par tout type de technique bien connu de l'homme du métier.

On aura de préférence recours à une clarification par filtration, décantation, ou encore centrifugation.

Dans le cas où la clarification est réalisée par centrifugation, on aura avantageusement recours à une centrifugation de l'extrait brut à une accélération comprise entre 8000 et 15000 g, de préférence 10.000 g et pendant un temps compris entre 3 et 10 min., et de manière tout à fait préférée pendant 5 minutes.

Une fois ces étapes préliminaires complétées, la protéine d'intérêt est isolée de la fraction surnageante de l'extrait par la mise en oeuvre des paramètres principaux du procédé de l'invention.

### EXEMPLES

### EXEMPLE 1: Procédé de purification de la lipase gastrique de chien recombinante à partir de grains de maïs.

### A) Réactifs

### 1) Produits chimiques

Les différents produits chimiques utilisés dans le procédé de purification de la lipase gastrique de chien recombinante (psl rDGL) sont au minimum de qualité analytique. Une liste de ces produits est détaillée ci-dessous:

| NOM | FOURNISSEUR | REFERENCE | REMARQUES |
|---|---|---|---|
| Tributyrine | Fluka | 91012 | |
| Sérum albumine bovine | Sigma | A-7906 | |
| Acide taurodésoxycholique | Sigma | T-0875 | sel de sodium (NaTDC) |
| NaCl | Merck | 10604.1000 | |
| NaOH | Merck | 9142.0500 | 0.02M |
| Acide bicinchoninique | Sigma | B-9643 | |
| Sulfate de cuivre | Sigma | C-2284 | |
| Sérum albumine bovine (BSA) | Sigma | P-0914 | solution étalon |
| Glycine | Merck | 4201.1000 | |
| HC | Merck | 100.317 | fumant |
| EDTA | Sigma | E-5134 | Sel disodique |
| Triton X100 | Sigma | X-100 | |
| DICB (terre de diatomées) | Meristem Therapeutics | | |
| Sulfate d'ammonium | Sigma | A-5132 | |
| Chlorure de magnésium | Sigma | M-8266 | Anhydre |
| Acétate de sodium | Merck | 106268 | Anhydre |
| Acétate d'ammonium | Sigma | A-7330 | |
| Acide acétique | Merck | 1.00062 | Glacial |
| NaOH | Merck | 1.05587.2500 | |
| Mannitol | Sigma | M-9647 | |

### 2) Solutions tampons.

Les tampons utilisés sont listés dans le tableau suivant :

| | | |
|---|---|---|
| Macération | Glycine-HCl 50 mM, pH 2,5 Chlorure de sodium 250m Triton X100 1 mM EDTA 1 mM | 14 volumes par rapport au poids de farine utilisée. |
| Lavage du clarcel | Glycine-HCl 50 mM, pH 2,5 Sulfate d'ammonium 40% (0,229 Kg/L) Triton X100 1 mM Chlorure de magnésium 75 mM | 7 volumes par rapport au poids de terre de diatomée |
| Désorption du clarcel | Glycine-HCl 50 mM, pH 2,5 Triton X100 1 mM Chlorure de magnésium 75 mM | 17 volumes par rapport au poids de terre de diatomée. |
| Dilution du rétentat | Glycine-HCl 50 mM, pH 3,0 | Qs suffisant pour conductivité adéquate |
| Equilibration concentré 10X SCHD | Glycine-HCl 500 mM, pH 3,0 | 7 volumes de colonne |
| Equilibration SCHD | Glycine-HCl 50 mM, pH 3,0 Chlorure de sodium 50 mM Triton X100 1 mM Chlorure de magnésium 75 mM | 7 volumes de colonne |
| Lavage 1, SCHD | Equilibration SCHD | 17 volumes de colonne |
| Lavage 2 SCHD | Glycine-HCl 50 mM, pH 3,0 Chlorure de sodium 50 mM Chlorure de magnésium 75 mM | 16 volumes de colonne |
| Elution SCHD | Acétate de sodium 50mM pH 4,0 Acide acétique 50mM Chlorure de sodium 500 mM | 8 volumes de colonne |
| Solutions de | NaCl 1M | 6 volumes de colonne |
| régénération SCHD | NaOH 500 mM | 6 volumes de colonne |
| Equilibration IMAC | Acétate de sodium 50 mM | 11 volumes de colonne |
| | pH 4,0 | |
| | Acide acétique 50mM | |
| | NaCl 500 mM | |
| Lavage 1 IMAC | Equilibration IMAC | 16 volumes de colonne |
| Sulfate de cuivre | Sulfate de cuiçvre 50 mM dans eau osmosée | 3,5 volumes de colonne |
| Lavage 2 IMAC | Glycine-HCl 10 mM pH 3,5 | 33 volumes de colonne |
| | Acétate d'ammonium 500 mM | |
| Elution IMAC | Glycine-HCl 10 mM pH 2,8 | 7 volumes de colonne |
| | Acétate d'ammonium 1 M | |
| Solutions de | HCl 1M | 5 volumes de colonne |
| régénération IMAC | NaOH 500 mM | 5 volumes de colonne |
| Diafiltration | Acide citrique-NaOH 20mM pH 4,0 | |

### B) Dosage des protéines

Les protéines sont dosées par la méthode à l'acide bicinchoninique (BCA) (Smith et al., Anal. Biochem. (1985), 150/ 76-85) La protéine de référence est une solution étalon à 1 mg/ml de BSA de chez Sigma. La mesure d'absorbance s'effectue sur microplaque 96 puits avec un lecteur IEMS/MF commercialisé par Labsystem, équipé d'un filtre interférentiel à 540 nm. Nombre d'analyses par lot : 4.

### C) Dosage d'activité de la lipase gastrique recombinante.

Le dosage d'activité de la lipase est effectué par titrimétrie au titrimètre de marque Mettler DL25 ou au titrimètre de marque Metrohm Titrino 718, à pH 5,0 et à 37°C sur tributyrine (Gargouri et al. Gastroenterology (1986), 91 : 919-925). Nombre d'analyses par lot : 4.

### D) Suivi du procédé de purification

Le procédé est contrôlé aux différentes étapes par dosage de l'activité sur tributyrine, dosage des protéines au BCA et estimation du pourcentage de pureté par analyse chromatographique en phase inverse sur colonne C4 (VYDAC, Colonne C4, 300 angströms, 250 mm X 4 mm)

Durant les étapes de purification par chromatographie, la détection des protéines s'effectue à 280 nm.

### E) Broyage

- 700 kg de maïs sont broyés avec un broyeur à impact de type Forplex.
- Le broyeur est refroidi par un courant d'azote liquide. Ainsi la température de la farine ne dépasse pas 20°C.

### F) Macération

Dans un premier temps, les conditions optimales retenues sont les suivantes:
Macération 1 :
Rapport plante/tampon : 1 + 8 (8 litres de tampon pour 1 kg de farine)
Durée de macération : 10H
Température : 20
Macération 2 :
Rapport plante/tampon : 1 + 2
Durée de macération : 2H
Température : 20 ± 2°C
Macération 3 :
Rapport plante/tampon : 1 + 4
Durée de macération : 8H
Température: 20 ± 2°C

### Mode opératoire :

- La farine est introduite manuellement dans une cuve inox de 10 800 litres, remplie au préalable avec un tampon de macération à pH 2,5.
- L'ensemble est mis sous agitation. Le pH du macérat 1 est alors de 3,0 ± 0,1, sinon il est nécessaire de l'ajuster à ce pH avec de l'acide chlorhydrique.
- Les boues obtenues après la première et la seconde décantation sont donc remises à macérer.
- Le volume de tampon de macération ajouté pour les macérations 2 et 3, est calculé à partir de la quantité de farine mise en oeuvre initialement.

Dans une variante du mode décrit ci-dessus, il est également possible de procéder de la manière suivante, ce qui permet de diminuer la durée de cette étape tout en maintenant le taux d'extraction de la lipase.

### Mode opératoire :

Macération 1 : 5 H puis décantation 1 (durée 15 H environ)
Dés l'apparition des boues issues de la décantation 1 : mise en macération 2
Macération 2 : 2H (début de décomptage à partir de la fin de la décantation 1) puis décantation 2 (durée 6H environ.)
Dés l'apparition des boues issues de la décantation 2 : mise en macération 3
Macération 3 : 8H (début de décomptage à partir de la fin de la décantation 2) puis décantation 3 (durée 10 H environ)

### G) Décantation

### Mode opératoire :

- Après chaque macération, le macérat obtenu est passé à l'aide d'une pompe à lobes dans un décanteur centrifuge dont le débit moyen est de 400 I/H.
- Les extraits bruts sont recueillis dans une cuve inox. Ils sont conservés à 4°C environ. L'extrait brut 2 est mélangé à l'extrait brut 1 . De même l'extrait brut 3,est mélangé aux extraits bruts 1 et 2 pour former l'extrait brut final EB dans une cuve inox de 10 800 litres agitée.

### H) Prépurification

1) Traitement au sulfate d'ammonium et adsorption sur terre de diatomée Mode opératoire :
   - Ajouter alors 0,164 Kg/L de sulfate d'ammonium après dissolution 1,5 % (p/v) de terre de diatomées (clarcel DIC B, en poids de clarcel par rapport au volume d'extrait brut).
   - L'ensemble est mis à agiter durant 30 ± 5 minutes dans une cuve à température ambiante.
2) Filtration, lavage et désorption sur terre de diatomée

### Mode opératoire :

Filtration
   - Après adsorption, l'agitation est arrêtée durant 1 heure.
   - Après adsorption, la suspension est filtrée sur un filtre contenant déjà une précouche effectuée avec 10 kg de clarcel
   - le filtrat obtenu (ne contenant pas la lipase) est éliminé
Lavage
   - Ensuite le lavage du gateau de filtration est réalisé en utilisant environ 7 volumes de tampon de lavage pour 1 kg de clarcel ayant servi à l'alluvionnage.
   - Le gâteau de clarcel est alors essoré.
Désorption
   - La désorption est réalisée avec environ 17 volumes de tampon de désorption pour 1 kg de clarcel engagé pour l'alluvionnage.
   - Le gâteau de clarcel est alors remis en suspension par mise en marche de l'agitation du filtre monoplaque. Après 30 minutes d'agitation, la suspension est filtrée.
   - Le filtrat obtenu est recueilli.

### I) Filtration

Deux types de filtration sont testées.

### 1) Filtration

### Mode opératoire :

- La fraction désorbée est filtrée sur des plaques K300 (plaque de cellulose contenant du Kieselghur et de la perlite) 40x40 cm de Seitz de porosité moyenne 10 µm.
- Le filtrat obtenu est recueilli dans une cuve.

Selon une variante de cette étape, on peut procéder comme décrit ci-dessous, ce qui permet de diminuer les problèmes techniques tels que colmatage, opacité des fractions, durée des opérations etc.

### 2) Désorption / Filtration

### Mode opératoire :

- Remettre en suspension le clarcel DIC B avec le tampon de désorption (17 Volumes /poids de clarcel), agiter durant 30 ± 5 minutes.
- Préparer le filtre Multiplaques en l'équipant de filtres K300.
- Filtrer le clarcel en suspension sur ce filtre multiplaques. A la fin de la filtration, pousser avec 2 volumes de tampon de désorption
- Recueillir la solution.

### J) Ultrafiltration

### Mode opératoire :

- Le filtrat est concentré à l'aide d'un système d'ultrafiltration, il est équipé de cartouches en polysulfone dont le seuil de coupure de la membrane est de 30 kd.
- Le rétentat est concentré quatre fois environ.

### K) Dilution

- Le rétentat stocké à 4°C environ est homogénéisé et dilué avec un tampon de dilution de façon à obtenir une conductivité égale ou supérieure de + 1 mS à celle du tampon d'équilibration SCHD.

### L) Chromatographie SCHD

La chromatographie SCHD correspond à une chromatographie d'échangeuse de cations, S-Ceramic-HyperD (Biosepra). La matrice de cette résine est constituée de silice céramisée et de dextran sur lequel sont greffés des groupements sulfonates ; La granulométrie des perles est de 60µm.
La chromatographie est réalisée à température ambiante.

### M) Chromatographie IMAC

La chromatographie IMAC est effectuée sur une résine dont la matrice est constituée de polyméthacrylate sur laquelle sont greffés des résidus d'acide iminodiacétique (fractogel EMD Chelate 650M, MERCK). Ces groupements favorisent la fixation d'ions métalliques, notamment de cuivre, qui fixent à leur tour la lipase par l'intermédiaire de leurs sites de coordination libres.
La granulométrie des perles est de 40 à 90µm.
La chromatographie est réalisée à température ambiante.

### N) Diafiltration

La fraction obtenue est diluée immédiatement avec deux volumes de tampons de diafiltration pour un volume fraction, puis est concentrée jusqu'à obtenir une concentration en protéines comprise entre 6 et 7 mg/ml, à l'aide d'un système d'ultrafiltration Millipore HUF/50 équipé de cartouches en polyethersulfone. Le seuil de coupure des membranes est de 30 kd. La température est comprise entre 17° et 19°C.

On réalise ensuite une diafiltration à une concentration de protéines constante jusqu'à obtenir une conductivité et un pH identiques à ceux du tampon de diafiltration.

### O) Lyophilisation

Le produit diafiltré est lyophilisé en vrac ou en flacons selon la méthode ci-après :
Après répartition de la solution par fractions aliquotes de 1 ml dans des flacons à sérum de marque Wheaton de 5 ml, les bouchons (gray butyle) sont posés sur les flacons sans les boucher et mis en place dans les boîtes boucheuses. Les boîtes boucheuses contenant les flacons sont introduites dans le lyophilisateur dont les étagères sont préalablement stabilisées à la température de - 45°C. Cette phase de congélation est maintenue pendant 3 h à pression atmosphérique. La pression est ensuite diminuée jusqu'à 0,15 mbar, puis la température des étagères est élevée à + 30°C avec une consigne de retour au bon vide (1 µbar) pour une valeur de -10°C. Le doseur cyclique d'énergie est placé à 5%, ce qui résulte en une vitesse de remontée en température de 5°C par heure. Entre 62 et 64 heures après le début de lyophilisation, la température des échantillons est stable et autorise la fin de l'opération de lyophilisation. Le vide est alors cassé par de l'azote C filtré sur un filtre de diamètre moyen de pore de 0,2 µm et les flacons sont bouchés puis sertis par des capsules en aluminium.

### EXEMPLE 2 : Procédé de purification de la lipase gastrique recombinante de chien à partir de feuilles de tabac transgénique.

Les matériels, produits chimiques, solutions tampons, filtres et supports chromatographiques sont identiques à ceux décrits pour l'exemple 1.

### A) Broyage des feuilles, clarification et adsorption sur terre de diatomées en présence de sulfate d'ammonium.

Les feuilles lyophilisées sont broyées à l'aide du (waring Blender) jusqu'à obtention d'une poudre.

La poudre est macérée pendant 15 min sous agitation lente dans du NaCl 0,2 M en maintenant le pH à 3 avec de l'HCl 1 M (30 ml de NaCl/g de poids sec).

Le produit homogénéisé est centrifugé à 10.000 g à + 4°C pendant 15 min. Le surnageant obtenu est filtré sur Miracloth.

Le produit homogénéisé est filtré et mis en contact avec de la terre de diatomées, puis avec du sulfate d'ammonium à 40 % de saturation. Une agitation est maintenue pendant 45 min.

Le mélange est filtré sur filtre papier 3Chr Whatman 20 µm et le gâteau restant est repris dans un tampon de lavage glycine/HCl 10 mM - NaCl 0,2 M-sulfate d'ammonium 40% pH 3 (1/5 volume HF+) et agité 5 min.

Le produit homogénéisé est filtré sur filtre papier 3Chr Whatman 20 µm et le gâteau est récupéré dans un tampon d'élution glycine/HCl 10 mM pH3 -NaCl 0,2 M (1 g de terre de départ/15 ml de tampon élution). Le mélange est agité 5 min puis filtré sur filtre papier 3Chr Whatman 20 µm.

### B) Chromatographie sur résine échangeuse de cations.

Le filtrat issu de l'étape A) est filtré sur 0,45 µm. L'échantillon est ensuite dilué avec du tampon citrate phosphate pH 3 (environ au 1/9) pour permettre d'ajuster la conductivité à celle du tampon d'équilibration de la colonne.

La chromatographie se déroule à 5°C dans les conditions décrites dans le tableau suivant:

**Protocole de chromatographie sur résine échangeuse de cations**

| Etapes | Solution | Débit (ml/min) | Nombre de volumes colonne |
|---|---|---|---|
| | Tampon citrate phosphate 20 mM pH 3 - NaCl 50 mM | 2 | 10 |
| Chargement | F2 dilué | 2 | |
| Lavage | Tampon citrate phosphate 20 mM pH 5,5 | 2 | 40 |
| | Tampon triéthanolamine | 2 | 15 |
| Elution | 20 mM pH 7 | 2 | 15 |

Les tampons citrate phosphate sont de l'acide citrique + sodium phosphate dibasique.

Tampon d'équilibration: 35 % phosphate et 63% d'acide citrique.

Tampon lavage: 63% phosphate et 28% d'acide citrique.

### C) Chromatographie sur résine IMAC-Cull

Cette étape est identique à l'étape M) de l'exemple 1.

Les étapes d'ultrafiltration, de diafiltration et de lyophilisation sont identiques aux étapes N) et O) décrites dans l'exemple 1.

**Tableau 1**

| Sulfate d'ammonium (% v/w) | Activité résiduelle (%) | Terre de diatomée (% w/v) |
|---|---|---|
| 0,00 | 100,28 | 0, 00 |
| 0,00 | 93,28 | 0,10 |
| 0,00 | 90,76 | 0,50 |
| 0,00 | 83,75 | 2,00 |
| 0,00 | 73,67 | 5,00 |
| 0,00 | 57,42 | 10,00 |
| 0,00 | 38,66 | 20,00 |
| 6,97 | 89,36 | 0.00 |
| 6,97 | 93,28 | 0,10 |
| 6,97 | 91,04 | 0,50 |
| 6,97 | 45,94 | 2,00 |
| 6,97 | 26,05 | 5,00 |
| 6,97 | 25,27 | 10,00 |
| 6,97 | 18,49 | 20,00 |
| 13,94 | 56,86 | 0,00 |
| 13,94 | 57,70 | 0,10 |
| 13,94 | 58,26 | 0,50 |
| 13,94 | 29,69 | 2,00 |
| 13,94 | 36,41 | 5,00 |
| 13,94 | 19,33 | 10,00 |
| 13,94 | 1,40 | 20,00 |
| 20,91 | 65,83 | 0,00 |
| 20,91 | 43,70 | 0,10 |
| 20,91 | 43,70 | 0,50 |
| 20,91 | 33,33 | 2,00 |
| 20,91 | 16,25 | 5,00 |
| 20,91 | 24,93 | 10,00 |
| 20,91 | 17,65 | 20,00 |
| 27,88 | 23,81 | 0,00 |
| 27,88 | 32,49 | 0,10 |
| 27,88 | 40,34 | 0,50 |
| 27,88 | 12,61 | 2,00 |
| 27,88 | 14,01 | 5,00 |
| 27,88 | 22,13 | 10,00 |
| 27,88 | 22,41 | 20,00 |
| 34,85 | 8,96 | 0,00 |
| 34,85 | 38,66 | 0,10 |
| 34,85 | 22,69 | 0,50 |
| 34,85 | 28,29 | 2,00 |
| 34,85 | 31,37 | 5,00 |
| 34,85 | 20,17 | 10,00 |
| 34, 85 | 14,85 | 20,00 |
| 41,82 | 14,01 | 0,00 |
| 41,82 | 19,89 | 0,10 |
| 41,82 | 24,93 | 0,50 |
| 41,82 | 24,09 | 2,00 |
| 41,82 | 34,73 | 5,00 |
| 41,82 | 28,29 | 10,00 |
| 41,82 | 9,80 | 20,00 |
| 48,80 | 7,84 | 0,00 |
| 48,80 | 11,48 | 0,10 |
| 48,80 | 3,64 | 0,50 |
| 48,80 | 15,97 | 2,00 |
| 48,80 . | 26,05 | 5,00 |
| 48,80 | 6,44 | 10,00 |
| 48,80 | 7,28 | 20,00 |

**Tableau 2**

| Sulfate de sodium (% v/w) | Activité résiduelle (%) | Terre de diatomée (% w/v) |
|---|---|---|
| 0,00 | 100,13 | 0,00 |
| 0,00 | 100,26 | 0,10 |
| 0,00 | 66,41 | 0,50 |
| 0,00 | 48,51 | 2,00 |
| 0,00 | 37,74 | 5,00 |
| 0,00 | 27, 37 | 10,00 |
| 0,00 | 23,35 | 20,00 |
| 1,00 | 100,78 | 0,00 |
| 1,00 | 66,54 | 0,10 |
| 1,00 | 51,88 | 0,50 |
| 1,00 | 42,80 | 2,00 |
| 1,00 | 39,17 | 5,00 |
| 1,00 | 28,79 | 10,00 |
| 1,00 | 21,53 | 20,00 |
| 2,00 | 63,81 | 0,00 |
| 2,00 | 71,08 | 0,10 |
| 2,00 | 51,36 | 0,50 |
| 2,00 | 28,96 | 2,00 |
| 2,00 | 33,72 | 5,00 |
| 2,00 | 27,89 | 10,00 |
| 2,00 | 25,42 | 20,00 |
| 5,00 | 90,01 | 0,00 |
| 5,00 | 4,80 | 0,10 |
| 5,00 | 5,45 | 0,50 |
| 5,00 | 4,54 | 2,00 |
| 5,00 | 4,93 | 5,00 |
| 5,00 | 4,02 | 10,00 |
| 5,00 | 3,50 | 20,00 |
| 10,00 | 65,11 | 0,00 |
| 10,00 | 72,37 | 0,10 |
| 10,00 | 83,79 | 0,50 |
| 10,00 | 89,88 | 2,00 |
| 10,00 | 87,55 | 5,00 |
| 10,00 | 54,47 | 10,00 |
| 10,00 | 42,15 | 20,00 |
| 15,00 | 57,85 | 0,00 |
| 15,00 | 85,34 | 0,10 |
| 15,00 | 90,92 | 0,50 |
| 15,00 | 91,96 | 2,00 |
| 15,00 | 82,10 | 5,00 |
| 15,00 | 64,85 | 10,00 |
| 15,00 | 34,24 | 20,00 |
| 20,00 | 49,16 | 0,00 |
| 20,00 | 53,18 | 0,10 |
| 20,00 | 66,02 | 0,50 |
| 20,00 | 42,80 | 2, 00 |
| 20,00 | 37,48 | 5,00 |
| 20,00 | 29,18 | 10,00 |
| 20,00 | 12,06 | 20,00 |

## Revendications

1. Procédé d'isolement et/ou de purification d'une protéine d'intérêt en solution comprenant des étapes d'agrégation partielle et d'adsorption de ladite protéine sur un support solide, ladite étape d'agrégation partielle comprenant l'introduction dans ladite solution d'un agent précipitant qui génère des assemblages moléculaires de ladite protéine, de petite taille, non susceptible de décanter spontanément et qui sont adsorbés sur ledit support solide.

2. Procédé selon la revendication 1, **caractérisé en ce que** les assemblages moléculaires de ladite protéine sont substantiellement formés d'agrégats protéiques de petite taille restant en suspension dans la solution (micro-agrégats).

3. Procédé selon la revendication 1, **caractérisé en ce que** l'agrégation partielle et l'adsorption de ladite protéine sont simultanées.

4. Procédé selon l'une des revendications précédentes **caractérisé en ce que** la cinétique de l'étape d'agrégation partielle est modifiée par la cinétique de l'étape d'adsorption.

5. Procédé selon la revendication 4, **caractérisé en ce que** la cinétique d'adsorption favorise la formation des micro-agrégats et défavorise la formation de macro-agrégats.

6. Procédé selon l'une des revendications précédentes, **caractérisé en ce que** la protéine d'intérêt est isolée et/ou purifiée à partir d'un milieu complexe.

7. Procédé selon la revendication 6, **caractérisé en ce que** le milieu comprend ladite protéine et au moins un composé choisi parmi des composés protéiques, des composés lipidiques, des composés polysaccharidiques, des polyphénols, ou encore des pigments, seuls ou en combinaison.

8. Procédé selon l'une des revendications précédentes, **caractérisé en ce que** l'agent précipitant est ajouté dans la solution contenant ladite protéine simultanément ou après l'introduction dudit support d'adsorption.

9. Procédé selon la revendication 8, **caractérisé en ce que** l'agent précipitant est un composé de type polyalkylène glycol, ou un polyol ou un sucre ou un sel précipitant organique ou inorganique.

10. Procédé selon la revendication 9, **caractérisé en ce que** le composé de polyalkylène glycol consiste en un polyéthylène glycol.

11. Procédé selon la revendication 9, **caractérisé en ce que** le sel précipitant organique est de l'acétate d'ammonium.

12. Procédé selon la revendication 9, **caractérisé en ce que** le sel précipitant inorganique est le sulfate d'ammonium ou le sulfate de sodium.

13. Procédé selon la revendication 10, **caractérisé en ce que** la concentration de sulfate d'ammonium se situe entre 10% et 60% en poids/volume, notamment entre 15 et 45% en poids/volume.

14. Procédé selon l'une des revendications 1 à 6, **caractérisé en ce que** le support d'adsorption est un support solide non greffé de ligands spécifiques susceptibles d'interagir avec la protéine d'intérêt.

15. Procédé selon la revendication 14, **caractérisé en ce que** le support solide est choisi parmi les supports organiques ou inorganiques.

16. Procédé selon la revendication 15, **caractérisé en ce que** le support solide inorganique est choisi parmi les supports à base de silices d'alumine ou d'oxydes de métaux ou de terre de diatomées.

17. Procédé selon la revendication 15, **caractérisé en ce que** le support solide organique est choisi parmi les supports à base de dextrans, d'agarose, de polyacrylamide, de cellulose, de polystyrène divynyl benzène, de nylon ou de métacrylate.

18. Procédé selon l'une quelconque des revendications 1 à 17 **caractérisé en ce qu'**il comporte une étape de, désorption de la protéine d'intérêt du support solide en l'absence de l'agent précipitant.

19. Procédé selon la revendication 18, **caractérisé en ce qu'**il comporte une ou plusieurs étapes de purification sur un support de chromatographie de la protéine désorbée.

20. Procédé selon la revendication 19, **caractérisé en ce que** le support de chromatographie est un support de chromatographie d'échange d'ions, un support de chromatographie d'exclusion de taille, un support de chromatographie d'interaction hydrophobe, un support de chromatographie d'affinité sur ions métalliques immobilisés, un support de chromatographie par affinité, ou un support de chromatographie à haute performance en phase liquide (HPLC).

21. Procédé selon l'une des revendications 19 ou 20, **caractérisé en ce qu'**il comporte une étape de passage de la protéine d'intérêt, désorbée du support solide, sur un support chromatographique d'échange de cations.

22. Procédé selon la revendication 21 **caractérisé en ce qu'**il comporte une étape de chromatographie d'affinité sur ions métalliques immobilisés (IMAC) de l'éluat issu de l'étape de chromatographie selon l'une quelconque des revendications 19 à 21.

23. Procédé selon la revendication 22, **caractérisé en ce que** le support chromatographique IMAC est à base d'ions Cu II.

24. Procédé selon l'une quelconque des revendications 19 à 23, **caractérisé en ce que** la ou les étapes de chromatographie est (sont) suivie(s) par une ou plusieurs étapes d'ultrafiltration ou de diafiltration.

25. Procédé selon l'une quelconque des revendications 1 à 24, **caractérisé en ce qu'**il comporte en outre une étape de séchage par lyophilisation ou atomisation de la protéine purifiée.

26. Procédé selon l'une quelconque des revendications 1 à 25, **caractérisé en ce que** la protéine d'intérêt est initialement contenue dans du matériel d'origine animale, bactérienne, fongique ou virale.

27. Procédé selon l'une quelconque des revendications 1 à 25, **caractérisé en ce que** la protéine d'intérêt est initialement contenue dans du matériel végétal.

28. Procédé selon la revendication 27, **caractérisé en ce que** le matériel végétal est un végétal oléagineux, protéagineux et/ou riche en polysaccharides et/ou en polyphénols et/ ou en pigments.

29. Procédé selon la revendication 28, **caractérisé en ce que** le matériel végétal est choisi parmi le maïs, le tabac, la tomate, le colza, le soja, le riz, la pomme de terre, la carotte, le blé, l'orge le tournesol, la laitue ou encore l'avoine.

30. Procédé selon l'une quelconque des revendications 27 à 29, **caractérisé en ce qu'**il comporte une première étape de broyage des graines ou de hachage des feuilles, suivi d'une étape de clarification par filtration ou centrifugation.

31. Procédé selon l'une quelconque des revendications 1 à 30, **caractérisé en ce que** la protéine d'intérêt est une lipase gastrique recombinante.

32. Procédé selon l'une quelconque des revendications 1 à 30, **caractérisé en ce que** la protéine d'intérêt est une lipase gastrique de chien recombinante.

## Patentansprüche

1. Verfahren zur Isolierung und/oder Reinigung eines in Lösung befindlichen Proteins von Interesse umfassend die Schritte partieller Aggregation und Adsorption des Proteins an einen festen Träger, wobei der Schritt der partiellen Aggregation das Einbringen eines präzipitierenden Agens in die Lösung umfasst, das molekulare Verbindungen des Proteins in kleiner Größe erzeugt, nicht geeignet zum spontanen Dekantieren, und die an den festen Träger adsorbiert werden.

2. Verfahren nach Anspruch 1, **dadurch gekennzeichnet, dass** die molekularen Verbindungen des Proteins substanziell aus Proteinaggregaten kleiner Größe gebildet werden, die in Suspension in der Lösung verbleiben (Mikroaggregate).

3. Verfahren nach Anspruch 1, **dadurch gekennzeichnet, dass** die partielle Aggregation und die Adsorption des Proteins gleichzeitig erfolgen.

4. Verfahren nach einem der vorhergehenden Ansprüche, **dadurch gekennzeichnet, dass** die Kinetik des Schritts der partiellen Aggregation durch die Kinetik des Schritts der Adsorption modifiziert wird.

5. Verfahren nach Anspruch 4, **dadurch gekennzeichnet, dass** die Adsorptionskinetik die Bildung von Mikroaggregaten begünstigt und die Bildung von Makroaggregaten benachteiligt.

6. Verfahren nach einem der vorhergehenden Ansprüche, **dadurch gekennzeichnet, dass** das Protein von Interesse aus einem komplexen Medium isoliert und/oder gereinigt wird.

7. Verfahren nach Anspruch 6, **dadurch gekennzeichnet, dass** das Medium das Protein und mindestens eine Verbindung ausgewählt aus Proteinverbindungen, Lipidverbindungen, Polysaccharidverbindungen, Polyphenolverbindungen oder des Weiteren Pigmente, alleine oder in Kombination, umfasst.

8. Verfahren nach einem der vorhergehenden Ansprüche, **dadurch gekennzeichnet, dass** das präzipitierende Agens der Lösung, die das Protein enthält, gleichzeitig oder nach Einbringen des Adsorptionsträgers zugegeben wird.

9. Verfahren nach Anspruch 8, **dadurch gekennzeichnet, dass** das präzipitierende Agens eine Verbindung des Typs Polyalkylenglycol oder ein Polyol oder ein Zucker oder ein präzipitierendes organisches oder anorganisches Salz ist.

10. Verfahren nach Anspruch 9, **dadurch gekennzeichnet, dass** die Polyalkylenglycolverbindung aus einem Polyethylenglycol besteht.

11. Verfahren nach Anspruch 9, **dadurch gekennzeichnet, dass** das präzipitierende organische Salz Ammoniumacetat ist.

12. Verfahren nach Anspruch 9, **dadurch gekennzeichnet, dass** das präzipitierende anorganische Salz Ammoniumsulfat oder Natriumsulfat ist.

13. Verfahren nach Anspruch 10, **dadurch gekennzeichnet, dass** sich die Konzentration des Ammoniumsulfats zwischen 10 und 60 Gewichts-/Volumenprozent, insbesondere zwischen 15 und 45 Gewichts-/Volumenprozent befindet.

14. Verfahren nach einem der Ansprüche 1 bis 6, **dadurch gekennzeichnet, dass** der Adsorptionsträger ein fester Träger ist, der nicht durch spezifische Liganden verändert ist, die geeignet sind, mit dem Protein von Interesse zu interagieren.

15. Verfahren nach Anspruch 14, **dadurch gekennzeichnet, dass** der feste Träger aus organischen oder anorganischen Trägern ausgewählt ist.

16. Verfahren nach Anspruch 15, **dadurch gekennzeichnet, dass** der feste anorganische Träger ausgewählt ist aus Trägern basierend auf Aluminiumsiliziumdioxid oder Metalloxiden oder Kieselgur.

17. Verfahren nach Anspruch 15, **dadurch gekennzeichnet, dass** der feste organische Träger ausgewählt ist aus Trägern basierend auf Dextranen, Agarose, Polyacrylamid, Cellulose, Polystyrendivinylbenzen, Nylon oder Metacrylat.

18. Verfahren nach einem der Ansprüche 1 bis 17, **dadurch gekennzeichnet, dass** es einen Schritt der Desorption des Proteins von Interesse von dem festen Träger in Abwesenheit des präzipitierenden Agens aufweist.

19. Verfahren nach Anspruch 18, **dadurch gekennzeichnet, dass** es einen oder mehrere Reinigungsschritte des desorbierten Proteins auf einem Chromatographieträger aufweist.

20. Verfahren nach Anspruch 19, **dadurch gekennzeichnet, dass** der Chromatographieträger ein Träger für lonenaustauschchromatographie, ein Träger für Größenausschlusschromatographie, ein Träger für Chromatographie durch hydrophobe Interaktionen, ein Träger für Affinitätschromatographie mittels immobilisierter Metallionen, ein Träger für Affinitätschromatographie oder ein Träger für Hochleistungsflüssigchromatographie (HPLC) ist.

21. Verfahren nach einem der Ansprüche 19 oder 20, **dadurch gekennzeichnet, dass** es einen Schritt des Durchlaufs des Proteins von Interesse, desorbiert von dem festen Träger, durch einen Kationenaustauschchromatographieträger aufweist.

22. Verfahren nach Anspruch 21, **dadurch gekennzeichnet, dass** es einen Affinitätschromatographieschritt mittels immobilisierter Metallionen (IMAC) für das Eluat, hervorgegangen aus einem Chromatographieschritt nach einem der Ansprüche 19 bis 21, aufweist.

23. Verfahren nach Anspruch 22, **dadurch gekennzeichnet, dass** der Chromatographieträger IMAC auf einem Kupfer II-Ion basiert.

24. Verfahren nach einem der Ansprüche 19 bis 23, **dadurch gekennzeichnet, dass** der oder die Chromatographieschritte gefolgt ist (sind) von einem oder mehreren Ultrafiltrations- oder Diafiltrationsschritten.

25. Verfahren nach einem der Ansprüche 1 bis 24, **dadurch gekennzeichnet, dass** es zudem einen Trocknungsschritt durch Lyophilisieren oder Atomisieren des gereinigten Proteins aufweist.

26. Verfahren nach einem der Ansprüche 1 bis 25, **dadurch gekennzeichnet, dass** das Protein von Interesse ursprünglich in einem Material tierischen, bakteriellen, Pilz- oder viralen Ursprungs enthalten ist.

27. Verfahren nach einem der Ansprüche 1 bis 25, **dadurch gekennzeichnet, dass** das Protein von Interesse ursprünglich in Pflanzenmaterial erhalten ist.

28. Verfahren nach Anspruch 27, **dadurch gekennzeichnet, dass** das Pflanzenmaterial eine ölhaltige, proteinhaltige Pflanze ist und/oder reich an Polysacchariden und/oder an Polyphenolen und/oder an Pigmenten ist.

29. Verfahren nach Anspruch 28, **dadurch gekennzeichnet, dass** das Pflanzenmaterial ausgewählt ist aus Mais, Tabak, Tomate, Raps, Soja, Reis, Kartoffel, Karotte, Weizen, Gerste, Sonnenblume, Blattsalat oder des Weiteren Hafer.

30. Verfahren nach einem der Ansprüche 27 bis 29, **dadurch gekennzeichnet, dass** es einen ersten Schritt des Zermalens von Samen oder des Schneidens von Blättern, gefolgt von einem Klärschritt durch Filtration oder Zentrifugation aufweist.

31. Verfahren nach einem der Ansprüche 1 bis 30, **dadurch gekennzeichnet, dass** das Protein von Interesse eine rekombinante gastrische Lipase ist.

32. Verfahren nach einem der Ansprüche 1 bis 30, **dadurch gekennzeichnet, dass** das Protein von Interesse eine rekombinante gastrische Lipase eines Hundes ist.

## Claims

1. Process for the isolation and/or purification of a protein of interest in solution comprising steps involving partial aggregation and adsorption of said protein onto a solid support, the step of partial aggregation comprising the introduction of a precipitating agent into said solution, which precipitating agent generates small size molecular assemblies of said protein, that are not susceptible to spontaneous decantation and that are adsorbed onto said solid support.

2. Process according to claim 1, **characterised in that** the molecular assemblies of said protein are substantially made up of small size protein aggregates (micro-aggregates) that remain in suspension in the solution.

3. Process according to claim 1, **characterised in that** the partial aggregation step and adsorption step of said protein are simultaneous.

4. Process according to any one of the preceding claims, **characterised in that** the kinetics of the partial aggregation step are modified by the kinetics of the adsorption step.

5. Process according to claim 4, **characterised in that** the kinetics of adsorption favour formation of the micro-aggregates and disadvantage formation of macro-aggregates.

6. Process according to any one of the preceding claims, **characterised in that** the protein of interest is isolated and/or purified from a complex media.

7. Process according to claim 6, **characterised in that** the media comprises said protein and at least one compound selected among protein compounds, lipid compounds, polysaccharide compounds, polyphenols, or even pigments, alone or in combination.

8. Process according to any one of the preceding claims, **characterised in that** the precipitating agent is added to the solution containing said protein simultaneously or after introduction of the adsorption support.

9. Process according to claim 8, **characterised in that** the precipitating agent is a compound of the type known as a polyalkylene glycol, or a polyol or a sugar or an organic or inorganic precipitating salt.

10. Process according to claim 9, **characterised in that** the polyalkylene glycol compound consists of polyethylene glycol.

11. Process according to claim 9, **characterised in that** the organic precipitating salt is ammonium acetate.

12. Process according to claim 9, **characterised in that** the precipitating inorganic salt is ammonium sulphate or sodium sulphate.

13. Process according to claim 10, **characterised in that** the concentration of ammonium sulphate is comprised between 10% and 60% wt/vol, particularly between 15% and 45% by wt/vol.

14. Process according to any one of claims 1 to 6, **characterised in that** the adsorption support is a solid support that does not contain grafted specific ligands likely to interact with the protein of interest.

15. Process according to claim 14, **characterised in that** the solid support is selected from organic and inorganic supports.

16. Process according to claim 15, **characterised in that** the inorganic solid support is selected from aluminium silicate supports or metal oxides or diatomaceous earths.

17. Process according to claim 15, **characterised in that** the organic solid support is selected from dextran based supports, agarose based supports, polyacrylamide based supports, cellulose based supports, polystyrene divinylbenzene based supports, nylon based or methacrylate based supports.

18. Process according to any one claims 1 to 17, **characterised in that** it comprises a desorption step of the protein of interest from the support in the absence of precipitating agent.

19. Process according to claim 18, **characterised in that** it comprises one or more purification steps of the desorbed protein on a chromatographic support.

20. Process according to claim 19, **characterised in that** the chromatographic support is an ion exchange chromatographic support, a size exclusion chromatographic support, a hydrophobic interaction chromatographic support, an immobilised metallic ion affinity chromatographic support, an affinity chromatographic support, or a high performance liquid phase chromatographic support (HPLC).

21. Process according to any one of claims 19 or 20, **characterised in that** it comprises a step involving passing the protein of interest, desorbed from the solid support, onto a cation exchange chromatographic support.

22. Process according to claim 21, **characterised in that** it comprises an immobilised metallic ion affinity chromatography step (IMAC) on the eluate resulting from the chromatography step of any one of claims 19 to 21.

23. Process according to claim 22, **characterised in that** the chromatographic IMAC support is based on copper II ions.

24. Process according to any one of claims 19 to 23, **characterised in that** the chromatography step or steps is/are followed by one or more ultrafltration or diafiltration steps.

25. Process according to any one of claims 1 to 24, **characterised in that** it also comprises a drying step by freeze-drying or atomization of the purified protein.

26. Process according to any one of claims 1 to 25, **characterised in that** the protein of interest is initially contained in material of animal, bacterial, fungal or viral origin.

27. Process according to any one of claims 1 to 25, **characterised in that** the protein of interest is initially contained in plant material.

28. Process according to claim 27, **characterised in that** the plant material is an oleinaceous plant, proteinaceous plant, and/or rich in polysaccharides and/or in polyphenols and/or pigments.

29. Process according to claim 28, **characterised in that** the plant material is chosen from maize, tobacco, tomato, rape, soya, rice, potato, carrot, wheat, barley, sunflower, lettuce or oats.

30. Process according to any one of claims 27 to 29, **characterised in that** it comprises a first step involving grinding seed or chopping leaves, followed by a clarification step involving filtration or centrifugation.

31. Process according to any one of claims 1 to 30, **characterised in that** the protein of interest is a recombinant gastric lipase.

32. Process according to one or more of claims 1 to 30, **characterised in that** the protein of interest is a recombinant dog gastric lipase.
